# FASCICULE DE BREVET EUROPEEN

(11) **EP 2 480 227 B1**
(45) Date de publication et mention de la délivrance du brevet: **22.08.2018**
(21) Numéro de dépôt: 10757595.3
(22) Date de dépôt: 23.09.2010
(51) Int. Cl.: A61K 31/198, A61K 31/64, A61K 31/403, A61K 31/445, A61K 31/195, A61P 27/00

(54) **NEUROPROTECTION RETINIENNE PAR DES INHIBITEURS DES CANAUX IONIQUES REGULES PAR LA SOUS-UNITE SUR**
RETINA-NEUROPROTEKTION DURCH IONENKANALHEMMER, DIE DURCH DIE SULFONYLHARNSTOFFREZEPTOR-UNTEREINHEIT (SUR) REGULIERT WERDEN
RETINAL NEUROPROTECTION BY IONIC CHANNEL INHIBITORS REGULATED BY THE SUR SUBUNIT

(30) Priorité: 24.09.2009 EP 09305892
(43) Date de publication de la demande: 01.08.2012
(73) Titulaire: Assistance Publique Hôpitaux De Paris, 75004 Paris (FR)
(72) Inventeur: POLAK, Michel, 75019 Paris (FR); BERDUGO POLAK, Marianne, 75019 Paris (FR); BEHAR-COHEN, Francine, 75016 Paris (FR); CRISANTI LASSIAZ, Patricia, 75015 Paris (FR)
(74) Mandataire: Gallois, Valérie
(86) Numéro de dépôt international: PCT/EP2010/064040
(87) Numéro de publication internationale: WO 2011/036202

(56) Documents cités:
- WO-A-02/072146
- WO-A-03/076393
- WO-A-2008/089103
- KONNO ET AL: "2-(1-Hexyn-1-yl)adenosine-induced intraocular hypertension is mediated via K<+> channel opening through adenosine A2A receptor in rabbits" EUROPEAN JOURNAL OF PHARMACOLOGY, ELSEVIER BV, NL, vol. 518, no. 2-3, 22 août 2005 (2005-08-22), pages 203-211, XP005028046 ISSN: 0014-2999
- TETSUSHI KIMURA, HITOSHI TAKAGI, KIYOSHI SUZUMA, MIHORI KITA, DAISUKE WATANABE AND NAGAHISA YOSHIMURA: "Comparisons between the beneficial effects of different sulphonylurea treatments on ischemia-induced retinal neovascularization" FREE RADICAL BIOLOGY AND MEDICINE, vol. 43, no. 3, 3 mai 2007 (2007-05-03), pages 454-461, XP002552903
- "Drug-induced myopia" PRESCRIRE INTERNATIONAL, vol. 12, no. 63, février 2002 (2002-02), pages 200-202, XP009125517
- TELLER J; RASIN M; ABRAHAM F A: "Accommodation insufficiency induced by glybenclamide" ANNALS OF OPHTHALMOLOGY, vol. 21, juillet 1989 (1989-07), pages 275-276, XP009125522
- CATALINA HERNÁNDEZ-SÁNCHEZ, TERESA L. WOOD AND DEREK LEROITH: "Developmental and Tissue-Specific Sulfonylurea Receptor Gene Expression" ENDOCRINOLOGY, vol. 138, no. 2, 1997, pages 705-711, XP002553154

## Description

La présente invention relève du domaine de la médecine, et plus particulièrement de celui du traitement des pathologies rétiniennes

### ARRIERE-PLAN TECHNOLOGIQUE DE L'INVENTION

La rétine est une membrane d'environ 0,5 mm d'épaisseur qui tapisse la face interne de l'oeil. Elle perçoit un signal lumineux et le transforme en signal électrique grâce à la phototransduction qui s'opère dans les photorécepteurs rétiniens. Ce signal électrique sera ensuite transmis au cerveau via les cellules bipolaires et ganglionnaires rétiniennes, puis via le nerf optique. La rétine est, d'un point de vue embryologique et histologique, un prolongement du système nerveux central puisqu'elle se forme au cours de l'embryogénèse à partir de déformations latérales de l'ampoule neurale formant les vésicules optiques qui donneront plus tard les rétines. Ainsi, l'oeil, et plus particulièrement la rétine, est relié directement au cerveau par le nerf optique. Celui-ci est formé par les axones des cellules ganglionnaires rétiniennes qui transmettent l'information collectée par la rétine le long des voies optiques jusqu'au cortex occipital dans lequel cette information est intégrée.

Cliniquement, la rétine est le siège de nombreuses pathologies dont celles associées à l'ischémie rétinienne qui résulte en une dégénérescence cellulaire responsable de l'altération de la fonction visuelle. Ces troubles ischémiques peuvent être observés dans diverses rétinopathies et neuropathies optiques aigues telles que les occlusions artérielles ou veineuses de la rétine ou les contusions oculaires, ou chroniques telles que la dégénérescence maculaire liée a l'âge (DMLA), le glaucome, la rétinopathie diabétique ou encore la rétinopathie du prématuré. Le glaucome et la DMLA sont les deux principales causes de cécité dans les pays industrialisés et constituent un problème majeur de santé publique.

De nombreuses études ont démontré que des phénomènes d'excitotoxicité étaient en partie responsables des neuropathies rétiniennes ischémiques, notamment dans le cas du glaucome (Franzco, 2006). En effet, les troubles ischémiques entraînent une stimulation excessive des récepteurs du glutamate, un neurotransmetteur excitateur. Cette activation induit une surcharge calcique responsable de lésions et de mort cellulaires. L'implication des récepteurs ionotropes du glutamate dans la toxicité de l'ischémie a été confirmée par l'effet neuroprotecteur de leurs antagonistes (Lam et al., 1997 ; Yoon et al., 1989 ; Osborne et al., 1996 ; Tsukahara et al., 1992). L'excitotoxicité peut également être responsable de la dégénérescence des cellules rétiniennes dans des pathologies telles que le décollement de la rétine (Sherry and Townes-Anderson, 2000), dans des atteintes rétiniennes liées à des pathologies associées à un déficit vasculaire telles que la rétinopathie drépanocytaire, ou encore dans le cas d'un traitement chirurgical de la rétine au laser (photocoagulation).

A ce jour, de nombreuses molécules ont été testées pour traiter ou prévenir les pathologies associées à l'ischémie rétinienne ou à l'excitotoxicité rétinienne, comme par exemple, des antagonistes compétitifs et non compétitifs des récepteurs du glutamate (Lam et al., 1997 ; Yoon et al., 1989 ; Vorwerck et al., 1996) ; des piégeurs de radicaux libres (Celeci et al., 2002; Szabo et al., 2001) ; des antagonistes des récepteurs adrénergiques (Donello et al., 2001; Chao et al., 2001) ; des facteurs neurotrophiques (Fontaine et al., 2002) ou encore des inhibiteurs de la libération pré-synaptique du glutamate.

Cependant, le mode d'administration de certaines de ces molécules ainsi que leur activité sur les autres organes, sont susceptibles de générer des effets secondaires indésirables. De plus, leur efficacité à des doses utilisables en clinique reste, à ce jour, très insuffisante. Il subsiste donc un réel besoin de trouver de nouveaux composés permettant de traiter et/ou de prévenir ces pathologies.

### RESUME DE L'INVENTION

L'objectif de la présente invention est de fournir de nouveaux composés pouvant être utilisés dans le traitement et/ou la prévention de pathologies associées à l'ischémie rétinienne et/ou à l'excitotoxicité rétinienne.

L'objet de la présente invention est défini par les revendications.

La présente invention concerne tout d'abord un inhibiteur des canaux ioniques régulés par la sous-unité SUR (SulfonylUrea Receptor) pour une utilisation dans le traitement et/ou la prévention d'une pathologie associée à l'ischémie rétinienne et/ou à une excitotoxicité rétinienne.

L'inhibiteur est sélectionné dans le groupe constitué du glibenclamide, de l'acétohexamide, du carbutamide, du glibornuride, du chlorpropamide, du tolbutamide, du tolazamide, du glipizide, du gliquidone, du glyclopyramide, du glisoxepide et du glimepiride. De préférence, l'inhibiteur est le glibenclamide.

Selon un mode particulier de réalisation, l'inhibiteur est administré en combinaison avec une autre substance active, de façon simultanée ou séquentielle.

Selon un mode de réalisation, la pathologie associée à l'ischémie rétinienne et/ou à une excitotoxicité rétinienne est sélectionnée dans le groupe constitué du glaucome, des neuropathies optiques glaucomateuses sans hypertonie, de la dégénérescence maculaire liée à l'âge, des inflammations intraoculaires aigues ou chroniques (les uvéites, uvéo-rétinites, les choroïdites), des neuropathies optiques ischémiques ou toxiques, de l'endophtalmie, des rétinites infectieuses, de la rétinopathie diabétique, de la rétinopathie du prématuré, de la rétinopathie ischémique proliférante, des rétinites pigmentaires, des rétinopathies associées à une hémoglobinopathie, d'une photo-dégénérescence, d'un décollement de la rétine, des pathologies vasculaires rétiniennes et choroïdiennes (sténoses, thromboses et occlusions vasculaires), des hémorragies rétiniennes et/ou choroïdiennes, et des dégénérescences rétiniennes héréditaires ou acquises. De préférence, la pathologie associée à l'ischémie rétinienne et/ou à une excitotoxicité rétinienne est sélectionnée dans le groupe constitué du glaucome, la dégénérescence maculaire liée à l'âge, la rétinopathie diabétique, la rétinopathie du prématuré, la rétinopathie ischémique proliférante et une rétinite pigmentaire.

Selon un mode particulier de réalisation, le sujet à traiter est un humain, en particulier un nourrisson, un enfant ou un adulte. Selon un autre mode de réalisation le sujet à traiter est un animal sélectionné dans le groupe constitué d'un chien, un chat, un cheval, une vache, un mouton, un cochon et un primate non-humain.

Selon un autre mode particulier de réalisation, l'inhibiteur est administré par voie intra-vitréenne, par voie sous-conjonctivale, par voie orale, par instillation topique, par voie péri-oculaire et intraoculaires ou par voie parentérale. De préférence, l'inhibiteur est administré par voie intra-vitréenne, par voie sous-conjonctivale, par voie orale ou par instillation topique

Dans un second aspect, le présent document décrit l'utilisation d'un inhibiteur des canaux ioniques régulés par la sous-unité SUR pour la fabrication d'un médicament destiné au traitement et/ou à la prévention d'une pathologie oculaire associée à l'ischémie rétinienne et/ou à une excitotoxicité rétinienne.

Dans un autre aspect, le présent document décrit une méthode de traitement et/ou de prévention d'une pathologie oculaire associée à une ischémie rétinienne et/ou à une excitotoxicité rétinienne chez un sujet, ladite méthode comprenant l'administration d'une dose thérapeutiquement efficace d'un inhibiteur des canaux ioniques régulés par la sous-unité SUR audit sujet.

### BREVE DESCRIPTION DES DESSINS

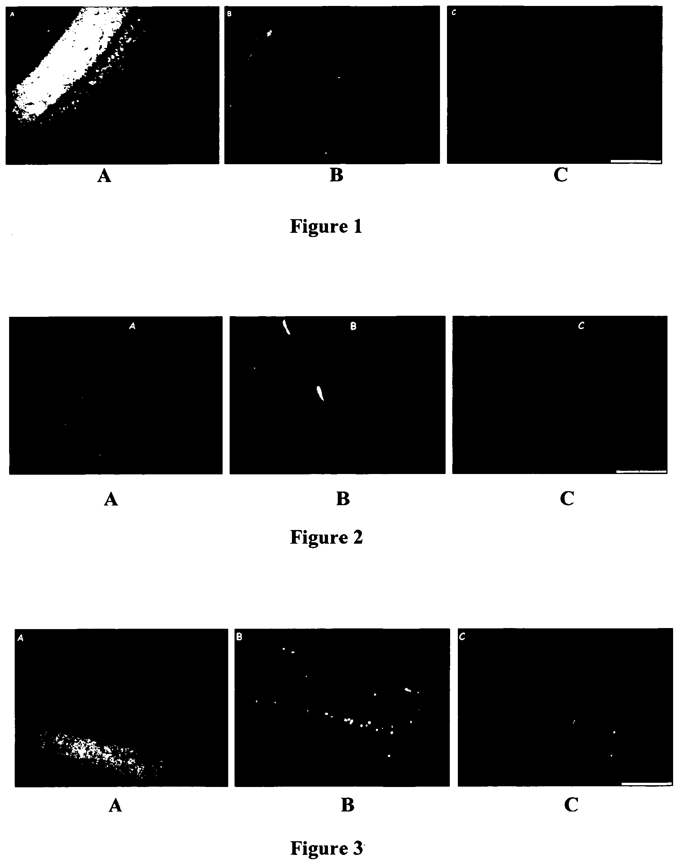

**Figure 1** **:** Contrôle négatif effectué sans enzyme TUNEL sur une lame de rétine témoin. (A) Marquage de la lame de rétine au DAPI. (B) Marquage de la lame de rétine au TUNEL. (C) Photos A et B fusionnées.
**Figure 2** : Contrôle positif effectué par observation de l'épithélium superficiel de la cornée des rétines étudiées, qui comporte habituellement des cellules apoptotiques. (A) Marquage au DAPI. (B) Marquage TUNEL. (C) Photos A et B fusionnées.
**Figure 3** : Dégénérescence des cellules de la rétine induite par le NMDA : couches des cellules bipolaires et ganglionnaires. (A) Marquage au DAPI. (B) Marquage au TUNEL. (C) Photos A et B fusionnées.

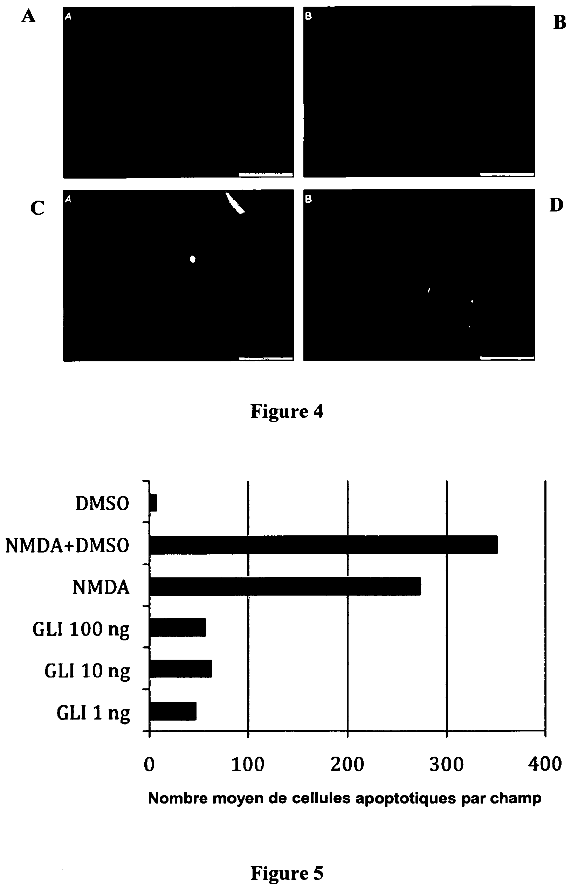

**Figure 4** : Effet du DMSO sur les cellules de la rétine avec ou sans injection de NMDA. (A) Marquage des cellules après injection de DMSO, au DAPI et au TUNEL. (B) Contrôle négatif effectué sans enzyme TUNEL. (C) Marquage au DAPI et au TUNEL des cellules de rétine après injection de DMSO avec NMDA. (D) Marquage au DAPI et au TUNEL des cellules de rétine, après injection de NMDA uniquement.
**Figure 5** **:** Histogramme des nombres moyens de cellules apoptotiques par champ pour chaque groupe de rats. DMSO : injection de DMSO seul (groupe 5) ; NMDA + DMSO : co-injection de NMDA et de DMSO (groupe 6) ; NMDA ; injection de NMDA seul (groupe 4) ; GLI 100 ng : co-injection de NMDA et de 100 ng de glibenclamide (groupe 3) ; GLI 10 ng : co-injection de NMDA et de 10 ng de glibenclamide (groupe 2) ; GLI 1 ng : co-injection de NMDA et de 1 ng de glibenclamide (groupe 1)

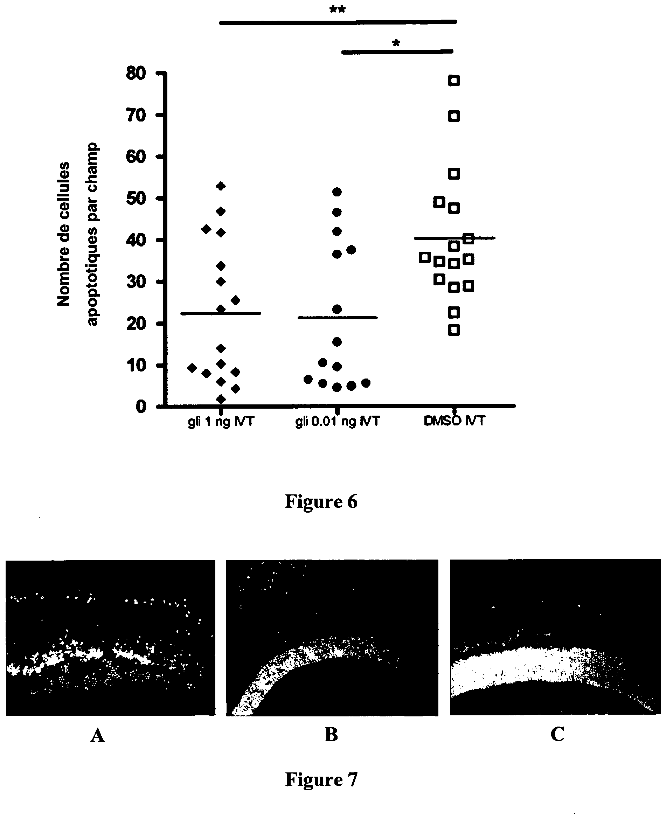

**Figure 6** **:** Graphique représentant le nombre de cellules apoptotiques par champ d'observation de coupe de rétine après injection intra-vitréenne de 1 ng de glibenclamide (gli 1 ng IVT), 0,01 ng de glibenclamide (gli 0,01 ng IVT) et d'une solution de DMSO (DMSO IVT). Test non paramétrique de Mann-Whitney (* P<0,05 ;** P<0,01 ;***P<0,001).
**Figure 7** **:** Structure des couches rétiniennes observées par marquage au DAPI. (A) après co-injection de NMDA et DMSO ; (B) après co-injection de NMDA et 1 ng de glibenclamide ; (C) après co-injection de NMDA et 0,01 ng de glibenclamide.

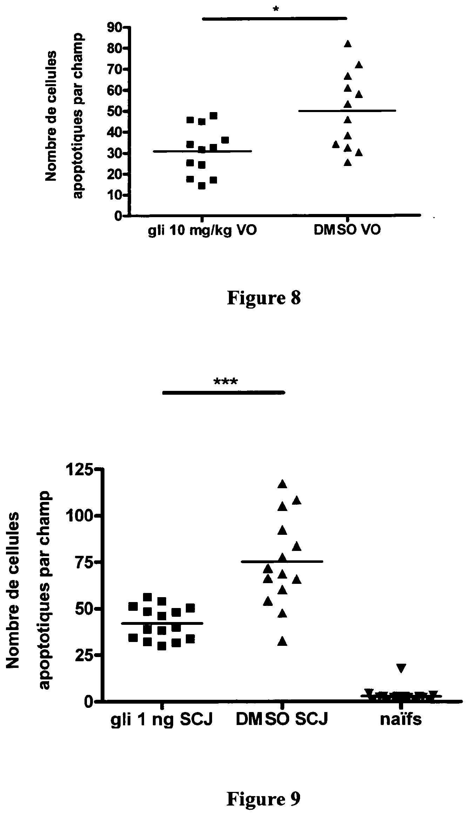

**Figure 8** **:** Graphique représentant le nombre de cellules apoptotiques par champ d'observation de coupe de rétine après administration par voie orale de 10 mg/kg de glibenclamide (gli 10 mg/kg VO) ou d'une solution de DMSO (DMSO VO) et injection intra-vitréenne de NMDA le même jour. Test non paramétrique de Mann-Whitney (* P=0,012).
**Figure 9** **:** Graphique représentant le nombre de cellules apoptotiques par champ d'observation de coupe de rétine après administration par voie sous-conjonctivale de 1 ng de glibenclamide (gli 1 ng SCJ) ou d'une solution de DMSO (DMSO SCJ) et injection intra-vitréenne de NMDA le même jour. Test non paramétrique de Mann-Whitney (*** P=0,0002).

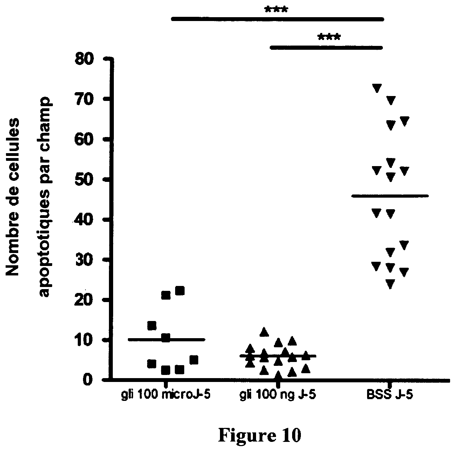

**Figure 10** **:** Graphique représentant le nombre de cellules apoptotiques par champ d'observation de coupe de rétine après administration par voie intra-vitréenne de 100 µg (gli 100 micro J-5) ou de 100 ng (gli 100 ng J-5) de glibenclamide ou d'une solution de BSS (BSS J-5) 5 jours avant l'injection intra-vitréenne de NMDA. Test non paramétrique de Mann-Whitney (*** P<0,0001).

### DESCRIPTION DETAILLEE DE L'INVENTION

Les canaux potassiques dépendants de l'ATP (K(ATP)) sont des hétéro-octomères formés de 4 sous-unités Kir6.x (inwardly rectifying K⁺ channel) délimitant le canal ionique lui-même, et de 4 sous-unités régulatrices SUR (sulfonylurea receptor). Ces canaux sont présents dans de nombreux types cellulaires, notamment dans les cellules pancréatiques et les cellules neuronales sous la forme Kir6.x/SURy.

Dans les cellules pancréatiques, les canaux Kir6.2/SUR1 sont impliqués dans la régulation glycémique. L'hyperglycémie sanguine provoque une augmentation de l'utilisation du glucose par la cellule bêta pancréatique, ce qui entraîne une production accrue d'ATP et une diminution du rapport ADP/ATP dans la cellule. La diminution de ce rapport induit la fermeture du canal K(ATP) Kir6.2/SUR1 conduisant à une dépolarisation cellulaire. Celle-ci induit l'ouverture du canal Ca²⁺ voltage dépendant, l'augmentation du Ca²⁺ intracellulaire et enfin la libération d'insuline.

Les sulfonylurées et les méglitinides sont des agents hypoglycémiants utilisés dans le traitement du diabète. Ils se lient à la sous-unité SUR des canaux Kir6.2/SUR1 et bloquent leur ouverture provoquant ainsi une libération accrue d'insuline.

Les canaux Kir6.x/SURy sont également présents dans le cerveau (Liss et al., 2001) où ils contribuent à la protection du système nerveux central (SNC) contre l'ischémie. En effet, l'activation de ces canaux est induite durant un état métabolique générant une déplétion de l'ATP, tel que l'hypoxie, et permet de réduire la consommation d'énergie. Cependant l'implication de ces canaux dans l'oedème cérébral consécutif à une ischémie a également été démontrée et l'utilisation de sulfonylurées a permis d'observer l'effet neuroprotecteur de ces composés dans un modèle d'ischémie cérébrale *in vitro* (Nistico et al., 2007).

Il a également été démontré que la sous-unité régulatrice SURI était surexprimée lors de lésions du SNC, notamment au cours de certaines ischémies cérébrales. Cette surexpression est corrélée à l'expression du canal calcique cation non-sélectif dépendant de l'ATP (NC_{Ca}-ATP) dont SURI constitue également la sous-unité régulatrice. En revanche, elle ne semble pas corrélée à l'expression du canal Kir6.2/SUR1 (Simard et al., 2006). Le canal NC_{Ca}-ATP n'est pas exprimé de manière constitutive dans le SNC mais uniquement lors de lésions neuronales ou d'épisodes ischémiques. L'administration de sulfonylurées lors d'une ischémie cérébrale permet, de par leur liaison à SURI, de réduire de moitié le taux de mortalité des patients mais également de réduire l'étendue des lésions (Simard et al., 2008 ; WO 2008/089103).

En revanche, l'action neuroprotectrice des sulfonylurées en tant qu'inhibiteurs des canaux ioniques, n'a jamais été démontrée dans d'autres tissus que le système nerveux central.

Dans la présente demande, les inventeurs ont mis en évidence que l'administration d'un inhibiteur des canaux ioniques régulés par la sous-unité SUR permettait de traiter ou de prévenir des pathologies rétiniennes associées à l'ischémie ou à des phénomènes d'excitotoxicité rétinienne ou de la myopie. Ils ont notamment démontré, dans un modèle *in vivo* de dégénérescence rétinienne, que ces inhibiteurs présentaient un effet neuroprotecteur rétinien et que leur utilisation permettait de diminuer les lésions rétiniennes engendrées par l'administration d'une excitotoxine telle que le N-méthyl-D-aspartate (NMDA). Les inventeurs ont en outre montré que ces inhibiteurs pouvaient être efficacement administrés par différentes voies, notamment par voie intra-vitréenne, orale ou sous-conjonctivale, et qu'ils étaient totalement dépourvus de toxicité vis-à-vis des cellules de la rétine, et ce, même administrés à forte dose.

Dans un premier aspect, la présente invention concerne un inhibiteur des canaux ioniques régulés par la sous-unité SUR pour une utilisation dans le traitement et/ou la prévention d'une pathologie oculaire associée à l'ischémie rétinienne et/ou à une excitotoxicité rétinienne.

Dans le présent document, le terme « inhibiteur des canaux ioniques régulés par la sous-unité SUR » se réfère à un composé capable de bloquer l'ouverture des canaux ioniques régulés par la sous-unité SUR et ainsi capable de bloquer le flux d'ions au travers de la membrane cellulaire, en se liant à une sous-unité desdits canaux. Les canaux ioniques régulés par la sous-unité SUR comprennent, par exemple, les canaux potassiques Kir6.2/SUR1, les canaux calciques cation non-sélectif dépendant de l'ATP (NC_{Ca}-ATP) et tout canal permettant une conductance ionique, couplé à la sous-unité SURI ou SUR2 (A ou B).

Selon un aspect décrit dans le présent document, l'inhibiteur des canaux ioniques régulés par la sous-unité SUR est un ligand de la sous-unité SUR. De préférence, la sous-unité SUR est sélectionnée dans le groupe constitué de SURI (GeneID : 6833), SUR2A et SUR2B (GeneID : 10060; tous deux codés par le gène ABCC9 (ATP-binding cassette, sub-family C (CFTR/MRP), member 9, aussi appelé ABC37, CMD1O, FLJ36852, SUR2) et issus d'épissage alternatif). De manière particulièrement préférée, la sous-unité SUR est la sous-unité SURI.

Selon un aspect décrit ici, l'inhibiteur des canaux ioniques régulés par la sous-unité SUR est sélectionné dans un groupe constitué des sulfonylurées et des méglitinides (ou glinides), et d'une combinaison quelconque de ceux-ci.

Les sulfonylurées susceptibles d'être utilisées selon la présente divulgation comprennent, à titre d'exemple et de façon non limitative, le glibenclamide (ou glyburide), l'acétohexamide, le carbutamide, le glibornuride, le chlorpropamide, le tolbutamide, le tolazamide, le glipizide, le gliclazide, le gliquidone, le glyclopyramide, le glisoxepide ou le glimepiride.

Les méglitinides susceptibles d'être utilisés selon la présente divulgation comprennent, à titre d'exemple et de façon non limitative, le répaglinide, le natéglinide ou le mitiglinide.

L'inhibiteur des canaux ioniques régulés par la sous-unité SUR est une sulfonylurée sélectionnée dans le groupe constitué du glibenclamide, l'acétohexamide, le carbutamide, le glibornuride, le chlorpropamide, le tolbutamide, le tolazamide, le glipizide, le gliquidone, le glyclopyramide, le glisoxepide ou le glimepiride. De manière particulièrement préférée, l'inhibiteur des canaux ioniques régulés par la sous-unité SUR est le glibenclamide.

L'inhibiteur des canaux ioniques régulés par la sous-unité SUR peut être utilisé seul ou en combinaison avec un ou plusieurs autres inhibiteurs desdits canaux.

L'inhibiteur des canaux ioniques régulés par la sous-unité SUR peut également être utilisé en combinaison avec une ou plusieurs autres substances actives. L'inhibiteur et ladite ou lesdites substances actives peuvent être administrés simultanément ou séquentiellement. Ces substances peuvent être notamment choisies de sorte à supprimer ou à limiter l'hypoglycémie provoquée par l'administration systémique d'agents hypoglycémiants tels que les sulfonylurées ou les méglitinides, comme par exemple du glucose ou du glucagon. Elles peuvent également être sélectionnées afin d'augmenter l'effet neuroprotecteur de la composition administrée. Dans ce cas, ces substances peuvent être choisies parmi les différentes substances connues pour leur activité de neuroprotection telles que, par exemple, la dizocilpine, la memantine, le riluzole, l'amantadine, le dextrométhorphane, le dextrorphane, le lacosamide, l'aptiganel, la brimonidine et les autres agonistes α-2adrénergiques, le diltiazem, les sirtuines et leurs activateurs tels que le resverastrol, la rapamycine et ses analogues, les agents dopaminergiques, les anti inflammatoires stéroïdiens et non stéroïdiens, en particulier l'acide acétyl-salicylique, les facteurs de croissance dits « insulin-like », en particulier l'insuline, l'IGF-1, l'IGF-2, l'hormone de croissance et leurs analogues, l'oestrogène et la progestérone et leurs analogues, les statines, les facteurs neurotrophiques en particulier le NGF, le PEDF, le GDNF, le CNTF, le VEGF, leurs analogues et leurs activateurs, les agents anti-oxydants capteurs de radicaux libres, ou encore les cellules souches et les cellules stromales de moelle osseuse. De manière alternative, ces substances peuvent être choisies parmi les différentes substances connues pour avoir des propriétés anti-ischémiques ou anti-excitotoxiques telles que, par exemple, la dizocilpine (MK 801), le magnésium, le dextrométhorphane, la flupirtine, la mémantine, la kétamine, la SOD/catalase, le lazaroïde, la diméthylthiourée, le diosmine/hespéridine, la mélatonine, la vitamine E, l'acide lipoïque, la déferoxamine, l'ibuprofène, le L-NMMA, le L-NNA, l'aminoguanidine, le L-NAME, la flunarizine, le ganglioside, le bétaxolol, la génistéine et l'ifenprodil.

L'inhibiteur selon l'invention peut également être administré en combinaison avec une ou plusieurs substances sélectionnées dans le groupe constitué par des béta-bloquants (par exemple du timolol), des agonistes alpha-2 adrénergiques (par exemple de la brimonidine), des analogues des prostaglandines F2 alpha (par exemple du latanoprost), des prostamides (par exemple le bimatoprost), des inhibiteurs de l'anhydrase carbonique par voie locale et/ou générale (par exemple du dorzolamine ou de l'acétazolamide), de l'adrénaline et des composés adrénaliniques, des anti-métabolites tels que la mitomycine C, le 5-fluorouracile ou le 5-chlorouracile, du thalidomide, des inhibiteurs des facteurs de croissance FGF, VEGF, PGF ou de leurs récepteurs, des inhibiteurs de l'IGF (insulin-like growth factor) de IRS-1 ou de IRS-2, des inhibiteurs de métalloprotéinases, des corticostéroïdes ou dérivés, des anti-inflammatoires non stéroïdiens, des inhibiteurs d'intégrines αvβ3 et αvβ5, des inhibiteurs de l'angiopoïétine, les statines (par exemple l'endostatine, l'angiostatine, l'octréotide), le carboxyamidotriazole, l'oestradiol et ses dérivés, des thrombospondines, des agents parasympathicolytiques, des agents sympathomimétiques, la sulfadiazine, la pyriméthamine, l'acide folinique, des agents anti-infectieux ou des agents anti-parasitaires (par exemple les hydroxynaphtoquinones), la vancomycine, l'amikacine, la ceftazidime, la gentamicine, des inhibiteurs de l'enzyme de conversion de l'angiotensine (par exemple le candésartan) ou des inhibiteurs ou des activateurs des protéine-kinases C, du carbogène et l'activateur tissulaire du plasminogène.

L'inhibiteur selon l'invention peut également être administré en combinaison avec une ou plusieurs substances utilisées pour traiter l'hypertension oculaire telles que des béta-bloquants (par exemple du timolol), des agonistes alpha-2 adrénergiques (par exemple de du brimonidine), des analogues des prostaglandines F2 alpha (par exemple du latanoprost), des prostamides (par exemple le bimatoprost), des inhibiteurs de l'anhydrase carbonique par voie locale et/ou générale (par exemple du dorzolamine ou de l'acétazolamide), de l'adrénaline et des composés adrénaliniques. L'inhibiteur selon l'invention peut également être administré à des patients traités pour l'hypertension oculaire par des moyens chirurgicaux tels que la trabéculectomie ou sclérectomie profonde, la trabéculoplastie au laser, un traitement par ultrasons ou cryochirurgie. L'inhibiteur selon l'invention peut également être administré en combinaison avec des anti-métabolites tels que la mitomycine C, le 5-fluorouracile ou le 5-chlorouracile.

L'inhibiteur selon l'invention peut également être administré en combinaison avec une ou plusieurs substances utilisées pour traiter ou inhiber la néovascularisation telles que le thalidomide, des inhibiteurs des facteurs de croissance FGF, VEGF, PGF ou de leurs récepteurs, des inhibiteurs de l'IGF (insulin-like growth factor) de IRS-1 ou de IRS-2, des inhibiteurs de métalloprotéinases, des corticostéroïdes ou dérivés, des anti-inflammatoires non stéroïdiens, des inhibiteurs d'intégrine αvβ3 et αvβ5, des inhibiteurs de l'angiopoïétine, les statines (par exemple l'endostatine, l'angiostatine, l'octréotide), le carboxyamidotriazole, l'oestradiol et ses dérivés, et les thrombospondines. L'inhibiteur selon l'invention peut également être administré à des patients soumis à un traitement chirurgical pour inhiber la néovascularisation tel que le traitement au laser, la photothérapie dynamique, la chirurgie, la radiothérapie ou la thermothérapie transpupillaire.

L'inhibiteur selon l'invention peut également être utilisé dans le traitement de l'uvéite en combinaison avec une ou plusieurs substances utilisées pour traiter l'uvéite. En particulier, il peut être utilisé en combinaison avec des corticoïdes, des anti-inflammatoires non stéroïdiens, des agents parasympathicolytiques ou des agents sympathomimétiques.

L'inhibiteur selon l'invention peut également être utilisé dans le traitement des rétino-choroïdites en combinaison avec une ou plusieurs substances utilisées pour traiter ces pathologies telles que les corticoïdes, la sulfadiazine, la pyriméthamine, l'acide folinique, des agents anti-infectieux ou des agents anti-parasitaires (par exemple les hydroxynaphtoquinones).

L'inhibiteur selon l'invention peut également être utilisé dans le traitement de l'endophtalmie en combinaison avec une ou plusieurs substances utilisées pour traiter cette pathologie telles que la vancomycine, l'amikacine, la ceftazidime, la gentamicine ou les corticoïdes. Il peut également être administré à des patients soumis à une vitrectomie.

L'inhibiteur selon l'invention peut également être administré à des patients soumis à un traitement chirurgical pour traiter la rétinopathie diabétique tel qu'un traitement des néovascularisations, la photocoagulation laser de l'oedème maculaire diabétique, la vitrectomie ou la pose d'implants intravitréens de corticoïdes. Il peut également être utilisé en combinaison avec une ou plusieurs substances utilisées pour traiter la rétinopathie diabétique telles que par exemple des inhibiteurs de l'enzyme de conversion de l'angiotensine (par exemple le candésartan) ou des inhibiteurs ou des activateurs des protéine-kinases C.

L'inhibiteur selon l'invention peut également être administré à des patients soumis à un traitement chirurgical pour traiter la rétinopathie du prématuré tel que le traitement au laser, la cryothérapie, la vitrectomie ou le plissement scléral. Il peut également être utilisé en combinaison avec une ou plusieurs substances utilisées pour traiter la rétinopathie du prématuré telles que par exemple des agents anti-inflammatoires non stéroïdiens.

L'inhibiteur selon l'invention peut également être administré à des patients soumis à un traitement chirurgical pour traiter des sténoses, occlusions et thromboses rétiniennes tel que la ponction de chambre antérieure, l'endartérectomie carotidienne ou un traitement des néovascularisations. Il peut également être utilisé en combinaison avec une ou plusieurs substances utilisées pour traiter des sténoses, occlusions et thromboses telles que des corticoïdes ou du carbogène.

L'inhibiteur selon l'invention peut également être administré à des patients soumis à un traitement chirurgical pour traiter des hémorragies rétino-choroïdiennes tel que l'injection de gaz de tamponnement (par exemple du perfluorocarbone). Il peut également être utilisé en combinaison avec une ou plusieurs substances utilisées pour traiter des hémorragies rétino-choroïdiennes telles que l'activateur tissulaire du plasminogène (tPA).

L'inhibiteur selon l'invention peut également être utilisé en combinaison avec une thérapie génique des rétinites pigmentaires.

Lorsque l'inhibiteur selon l'invention est administré à des patients soumis à un traitement chirurgical, il peut être administré avant, pendant ou après le traitement chirurgical.

Tel qu'utilisé dans le présent document, le terme « traitement » se réfère à tout acte permettant de diminuer, de supprimer ou de retarder les symptômes associés à une pathologie. Le terme « prévention » se réfère à toute action destinée à prévenir l'apparition des symptômes associés à une pathologie. De façon plus particulière, le terme « traitement d'une pathologie oculaire associée à l'ischémie rétinienne et/ou à une excitotoxicité rétinienne » se réfère tout acte permettant de diminuer, de supprimer ou de retarder la dégénérescence des cellules rétiniennes. Le terme « prévention d'une pathologie oculaire associée à l'ischémie rétinienne et/ou à une excitotoxicité rétinienne » se réfère à toute action destinée à prévenir l'apparition de phénomène de dégénérescence des cellules rétiniennes.

Le terme « pathologie oculaire associée à l'ischémie rétinienne et/ou à une excitotoxicité rétinienne », tel qu'utilisé dans ce document, se réfère à tout état pathologique impliquant une atteinte rétinienne générée par un phénomène d'ischémie et/ou d'excitotoxicité. Ces pathologies comprennent, à titre d'exemple et de façon non limitative, le glaucome, les neuropathies optiques glaucomateuses sans hypertonie (telles que le glaucome à pression normale), la dégénérescence maculaire liée à l'âge, les inflammations intraoculaires aigues ou chroniques (les uvéites, uveo-rétinites, les choroïdites), les neuropathies optiques ischémiques ou toxiques, l'endophtalmie, les rétinites infectieuses, la rétinopathie diabétique, la rétinopathie du prématuré, la rétinopathie ischémique proliférante, les rétinites pigmentaires, les rétinopathies associées à une hémoglobinopathie telle que la drépanocytose ou la thalassémie, une photo-dégénérescence ou encore un décollement de la rétine, les pathologies vasculaires rétiniennes et choroïdiennes (sténoses, thromboses et occlusions), les hémorragies rétiniennes et/ou choroïdiennes, la myopie ou encore des dégénérescences rétiniennes héréditaires ou acquises.

Ces pathologies oculaires peuvent également provenir des effets secondaires de certains traitements ophtalmiques notamment les chirurgies oculaires, la photocoagulation, la photothérapie dynamique, la thermothérapie transpupillaire, l'injection intra-oculaire de gaz, de l'administration de certains médicaments tels que, par exemple, les interférons, les corticostéroïdes, les anti-angiogéniques, les agents vasoconstricteurs ou les chimio-thérapies ou encore de traitements radiothérapeutiques. Afin de prévenir ces pathologies, l'inhibiteur selon l'invention peut être administré préalablement et/ou simultanément et/ou ultérieurement au traitement thérapeutique susceptible d'engendrer une atteinte rétinienne.

Certaines pathologies oculaires impliquant une dégénérescence des cellules rétiniennes provoquée par un phénomène d'ischémie ou d'excitotoxicité peuvent également trouver leur origine dans les pathologies artérielles, veineuses ou microvasculaires, dans des anomalies hématologiques ou dans des hémorragies ou des infarctus.

Selon un mode particulier de réalisation, la pathologie associée à l'ischémie rétinienne et/ou à une excitotoxicité rétinienne est sélectionnée dans le groupe constitué du glaucome, des neuropathies optiques glaucomateuses sans hypertonie, de la dégénérescence maculaire liée à l'âge, la rétinopathie diabétique, la rétinopathie du prématuré, la rétinopathie ischémique proliférante et une rétinite pigmentaire.

Selon un autre mode particulier de réalisation, la pathologie associée à l'ischémie rétinienne et/ou à une excitotoxicité rétinienne est sélectionnée dans le groupe constitué des neuropathies optiques glaucomateuses sans hypertonie, les inflammations intraoculaires aigues ou chroniques (les uvéites, uveo-rétinites, les choroïdites), les neuropathies optiques ischémiques ou toxiques, l'endophtalmie, les rétinites infectieuses, la rétinopathie du prématuré, les rétinites pigmentaires, les rétinopathies associées à une hémoglobinopathie telle que la drépanocytose ou la thalassémie, une photo-dégénérescence ou encore un décollement de la rétine, les pathologies vasculaires rétiniennes et choroïdiennes (sténoses, thromboses et occlusions), les hémorragies rétiniennes et/ou choroïdiennes, ou encore des dégénérescences rétiniennes héréditaires ou acquises.

Selon un autre mode de réalisation particulier, la pathologie associée à l'ischémie rétinienne et/ou à une excitotoxicité rétinienne est sélectionnée dans le groupe constitué du glaucome à pression normale, les uvéites, les uvéo-rétinites, les choroïdites, les rétinites infectieuses, la rétinopathie du prématuré, les rétinites pigmentaires, les rétinopathies associées à la drépanocytose ou la thalassémie et les hémorragies rétiniennes et/ou choroïdiennes.

Selon un autre mode de réalisation particulier, la pathologie associée à l'ischémie rétinienne et/ou à une excitotoxicité rétinienne est la myopie.

Selon un mode de réalisation préféré, la pathologie associée à l'ischémie rétinienne et/ou à une excitotoxicité rétinienne est le glaucome ou la rétinopathie diabétique. Selon un mode de réalisation particulièrement préféré, la pathologie associée à l'ischémie rétinienne et/ou à une excitotoxicité rétinienne est le glaucome.

Selon un aspect décrit ici, la pathologie associée à l'ischémie rétinienne et/ou à une excitotoxicité rétinienne est la rétinopathie diabétique et l'inhibiteur des canaux ioniques régulés par la sous-unité SUR est sélectionné dans le groupe constitué du glibenclamide, l'acétohexamide, le carbutamide, le glibornuride, le chlorpropamide, le tolbutamide, le tolazamide, le glipizide, le gliquidone, le glyclopyramide, le glisoxepide, le glimepiride, le répaglinide, le natéglinide et le mitiglinide, et une combinaison quelconque de ceux-ci. De préférence, l'inhibiteur des canaux ioniques régulés par la sous-unité SUR est le glibenclamide.

Le sujet à traiter, ou patient, est un animal, de préférence un mammifère. Selon un mode de réalisation, le sujet à traiter est un animal sélectionné dans le groupe constitué d'un chien, un chat, un cheval, une vache, un mouton, un cochon et un primate non-humain. Selon un mode de réalisation préférée, le sujet à traiter est un humain sélectionné dans le groupe constitué par un nourrisson, un enfant et un adulte.

L'inhibiteur utilisé selon l'invention est administré au patient sous la forme d'une composition pharmaceutique comprenant au moins un inhibiteur des canaux ioniques régulés par la sous-unité SUR et un support et/ou excipient pharmaceutiquement acceptable. Les excipients et supports pharmaceutiquement acceptables susceptibles d'être utilisés sont bien connus de l'homme du métier (Remington's Pharmaceutical Sciences, 18th édition, A. R. Gennaro, Ed., Mack Publishing Company [1990]; Pharmaceutical Formulation Development of Peptides and Proteins, S. Frokjaer and L. Hovgaard, Eds., Taylor & Francis [2000]; and Handbook of Pharmaceutical Excipients, 3rd édition, A. Kibbe, Ed., Pharmaceutical Press [2000]). La composition pharmaceutique comprenant l'inhibiteur des canaux ioniques régulés par la sous-unité SUR peut être sous forme de comprimés, capsules, gélules, granulats, suspensions, émulsions, solutions, polymères, nanoparticules, microsphères, suppositoires, lavements, gels, pates, onguents, crèmes, emplâtres, potions, injectables, implants, sprays ou aérosols.

L'inhibiteur des canaux ioniques régulés par la sous-unité SUR utilisé selon la présente invention peut être administré par voie orale, sublinguale, cutanée, sous-cutanée, intramusculaire, intraveineuse, parentérale, topique, locale, intratrachéale, intranasale, transdermique, rectale, intra-vitréenne, intra-camérulaire, sous-rétinienne, supra-choroïdienne, intra-oculaire, péri-oculaire, sous-conjonctivale ou intra-auriculaire. De préférence, l'inhibiteur des canaux ioniques régulés par la sous-unité SUR est administré par voie intra-vitréenne, sous-conjonctivale ou orale ou par instillation topique. Le composé peut également être utilisé comme agent d'irrigation au cours de la chirurgie vitréo-rétinienne ou comme adjuvant d'une chirurgie intraoculaire. Il peut alors éventuellement être co-administré avec un agent de tamponnement interne dégradable ou non dégradable.

L'inhibiteur selon l'invention est administré au patient à une dose thérapeutiquement efficace. Le terme « dose thérapeutiquement efficace » tel qu'utilisé ici se réfère à la quantité d'inhibiteur des canaux ioniques régulés par la sous-unité SUR nécessaire pour observer une activité thérapeutique ou préventive sur une pathologie oculaire associée à une ischémie rétinienne et/ou à une excitotoxicité rétinienne. La quantité d'inhibiteur des canaux ioniques régulés par la sous-unité SUR à administrer ainsi que la durée du traitement sont évaluées par l'homme du métier selon l'état physiologique du sujet à traiter, la pathologie à traiter ou à prévenir, l'inhibiteur choisi et la voie d'administration utilisée. L'inhibiteur selon l'invention peut être administré sous la forme d'une dose unique ou de doses multiples.

Selon un mode de réalisation de l'invention, l'inhibiteur est administré au patient par voie intra-vitréenne sous la forme d'une composition pharmaceutique comprenant entre 0,01 pg et 100 µg d'inhibiteur par unité de dose, de préférence entre 0,01 ng et 1000 ng d'inhibiteur, et de manière particulièrement préférée entre 0,01 ng et 100 ng d'inhibiteur.

Selon un autre mode de réalisation, l'inhibiteur est administré au patient par voie orale sous la forme d'une composition pharmaceutique comprenant entre 0,001 mg et 150 mg d'inhibiteur par kilo de poids corporel par unité de dose, de préférence entre 0,01 mg et 150 mg d'inhibiteur par kilo de poids corporel, et de manière particulièrement préférée entre 5 et 15 mg d'inhibiteur par kilo de poids corporel. Selon encore un autre mode de réalisation, l'inhibiteur est administré au patient par voie sous-conjonctivale sous la forme d'une composition pharmaceutique comprenant entre 0,1 pg et 1000 ng d'inhibiteur par unité de dose, de préférence entre 0,1 ng et 100 ng d'inhibiteur, et de manière particulièrement préférée entre 1 ng et 100 ng d'inhibiteur.

Selon un mode de réalisation particulier, l'inhibiteur est administré au patient par voie intra-vitréenne sous la forme d'une composition pharmaceutique comprenant entre 0,1 pg et 1 pg d'inhibiteur par unité de dose, de une à 4 fois par mois. De préférence, le composé est formulé dans un système à libération prolongée permettant son injection toutes les 4 à 8 semaines. De façon particulièrement préférée, le composé est formulé dans un système à libération prolongée permettant son injection deux à quatre fois par an.

Le présent document décrit également l'utilisation d'un inhibiteur des canaux ioniques régulés par la sous-unité SUR pour la fabrication d'un médicament destiné au traitement et/ou à la prévention d'une pathologie oculaire associée à l'ischémie rétinienne et/ou à une excitotoxicité rétinienne.

Le présent document décrit en outre une méthode de traitement et/ou de prévention d'une pathologie oculaire associée à une ischémie rétinienne et/ou à une excitotoxicité rétinienne chez un sujet, ladite méthode comprenant l'administration d'une dose thérapeutiquement efficace d'un inhibiteur des canaux ioniques régulés par la sous-unité SUR audit sujet. Selon un aspect, le sujet à traiter est un animal sélectionné dans le groupe constitué d'un chien, un chat, un cheval, une vache, un mouton, un cochon et un primate non-humain. Selon un aspect préféré, le sujet à traiter est un humain.

D'autres caractéristiques et avantages de l'invention apparaîtront mieux à la lecture des exemples suivants donnés à titre illustratif et non limitatif.

### EXEMPLES

### Matériel et méthodes

### Modèle de dégénérescence rétinienne induite par le NMDA chez le rat

Pour induire une neurodégénérescence rétinienne, les inventeurs ont injecté dans le corps vitré des rats du N-methyl-D-aspartate (NMDA), modèle connu d'excitotoxicité rétinienne au cours duquel le NMDA, analogue synthétique du glutamate, contribue à la mort cellulaire (Crisanti et al., 2006). Présent en trop grande quantité, le NMDA hyperactive les récepteurs excitateurs neuronaux (les récepteurs NMDA) qui deviennent perméables au calcium. L'injection de NMDA provoque une augmentation de la concentration intracellulaire de Ca²⁺. Le calcium intracellulaire active des enzymes (phospholipases C, endonucléases, protéases) qui dégradent les structures cellulaires et conduisent à l'apoptose.

### Animaux

Des rats Wistar mâles âgés de 8 semaines pesant 320 à 350g ont été utilisés. Les rats sont maintenus dans une animalerie standard avec un cycle lumineux de 12h jour/12h nuit, sous une température de 21°C +/- 1°C et une humidité de 55 +/- 5%.

### Anesthésies

Les rats ont été anesthésiés par une injection intramusculaire de pentobarbital sodique (30 mg/kg, CEVA Santé Animale). Une anesthésie locale des yeux a été obtenue par instillations de Tétracaïne© (collyre, 1X, unidose 0,4 ml, laboratoire TVM, Lempdes, France).

### Couves en congélation

Les yeux énucléés des rats ont été placés dans des moules « cubiques » remplis d'un gel d'enrobage (Tissue Teck© OCT - Optimal Cutting Temperature) qui permet de congeler les yeux à -80°C lorsque l'ensemble du dispositif est plongé dans de l'azote liquide. Ces moules permettent d'obtenir des blocs de forme cubique afin d'obtenir par la suite des coupes nettes en évitant des mouvements involontaires des globes oculaires qui risqueraient d'altérer l'agencement des tissus qui forment l'oeil.

Les coupes ont été réalisées à - 20°C et mesuraient 10 µm d'épaisseur. Ces coupes ont été placées sur des lames préalablement gélatinées pour permettre leur adhérence.

### Méthode TUNEL

### (Tunel = TdT mediated dUTP-biotin Nick End Labeling; TdT = Terminal deoxynucleotidyl Transferase)

Cette technique est utilisée pour détecter les cellules apoptotiques. Elle est basée sur le fait que l'apoptose s'accompagne d'une fragmentation de l'ADN. L'enzyme TdT permet d'ajouter aux extrémités 3'-OH issues de la fragmentation de l'ADN un nucléotide marqué. Le signal est ensuite amplifié par une peroxydase.

Les coupes de rétines ont été perméabilisées par incubation dans une solution de Triton X100 (0,3%) pendant 20 minutes à température ambiante puis incubées à 37°C pendant 1 heure avec 50 µL par lame de mélange réactionnel TUNEL (mélange obtenu avec 40 µL d'enzyme TUNEL et 360µL de label TUNEL, solution contenant des nucléotides couplés au FITC).

Les coupes ont été lavées cinq fois dans du PBS 1X et une fois dans de l'eau distillée, montées sous lamelles et observées au microscope à fluorescence (pour le TUNEL-FITC : excitation à 488 nm et émission à 520 nm; pour le DAPI : excitation à 372 nm et émission à 456 nm).

La quantité de mort cellulaire par apoptose et le type de cellules concernées ont été évalués par le nombre et la localisation intra-rétinienne des noyaux positifs à la méthode TUNEL. Pour chaque oeil, quatre coupes complètes de rétine, prises dans les mêmes secteurs de l'oeil, ont servi au comptage des cellules apoptotiques. Le nombre total de cellules apoptotiques par rétine a été rapporté au nombre de champs comptés (donc à la longueur rétinienne qui varie d'un endroit à l'autre de la rétine). La moyenne de ces nombres par champ a été calculée pour une coupe entière de rétine (formée de 7 à 14 champs); et ces moyennes, calculées pour les différents groupes de traitements ont été comparées entre elles grâce au test non paramétrique de Mann Whitney. Les différences dont le P est <0,05 sont considérées comme significatives.

### Exemple 1 : effet du glibenclamide sur la dégénérescence rétinienne induite par NMDA

### Protocole expérimental

L'utilisation de diméthylsulfoxyde (DMSO) est nécessaire à la solubilisation du glibenclamide. Afin de réduire le nombre d'injections intravitréennes, le NMDA et le glibenclamide (5g, MPBIO® Europe, Illkirch, France) préalablement dissout dans du DMSO, ont été co-injectés dans une même solution, après avoir vérifié l'absence de précipitation du mélange aux concentrations utilisées. Le DMSO a été utilisé à la concentration minimale nécessaire à la dissolution du glibenclamide (260 mM).

Trois solutions de glibenclamide comprenant 1, 10 ou 100 ng de glibenclamide pour 3 µL de solution ont été préparées. Pour cela, une solution mère comprenant 9 mg de glibenclamide en poudre dissouts dans 900 µL de DMSO (concentration de 10 µg/µL de glibenclamide) a été diluée dans du PBS 1X de manière à obtenir les concentrations souhaitées.

Selon le protocole expérimental présenté ci-dessous, dans les groupes tests, le glibenclamide et le NMDA ont été co-injectés en mélangeant 3 µL de solution de glibenclamide (comprenant 1, 10 ou 100 ng de glibenclamide) et 3µL de solution de 90 nM NMDA (soit une concentration finale dans la solution injectée de 45 nM NMDA).

Le protocole expérimental comprenait six groupes de rats tels que définis ci-dessous
Groupe 1 : (n=2, 4 yeux), injection d'une solution de 6µL contenant 45 nM de NMDA, 1 ng de glibenclamide et 260 mM de DMSO.
Groupe 2 : (n=2, 4 yeux), injection d'une solution de 6µL contenant 45 nM de NMDA, 10 ng de glibenclamide et 260 mM de DMSO.
Groupe 3 : (n=2, 4 yeux), injection d'une solution de 6µL contenant 45 nM de NMDA, 100 ng de glibenclamide et 260 mM de DMSO.
Groupe 4 : (n=2, 4 yeux), injection d'une solution de 6µL contenant uniquement 45 nM de NMDA dans du tampon PBS 1X (groupe contrôle).
Groupe 5 : (n=2, 4 yeux), injection d'une solution de 6µL contenant uniquement 260 mM de DMSO dans du tampon PBS 1X (groupe contrôle).
Groupe 6 : (n=2, 4 yeux), injection d'une solution de 6µL contenant 45 nM de NMDA et 260 mM de DMSO (groupe contrôle).

24h après les injections intra-vitréennes, les rats ont été euthanasiés par inhalation de CO₂ (1 minute). Les yeux ont été immédiatement énucléés et brièvement nettoyés dans du PBS 1X avant d'être percés à l'aide d'une aiguille 20G pour faire pénétrer le paraformaldéhyde. La fixation des yeux a été réalisée durant 1 nuit à 4°C dans 4% de paraformaldéhyde dilué dans du PBS 1X. Après fixation, les yeux ont ensuite été plongés dans une solution contenant 20% de sucrose dilué dans du PBS 1X, jusqu'à flottaison (soit environ 2 heures). Cette étape a pour but de prévenir les altérations des tissus provoquées par la congélation brutale.

Les yeux ont ensuite subi une étape d'inclusion en OCT, une étape de coupe en congélation au cryostat (10µm d'épaisseur) et une étape de marquage des coupes par la méthode TUNEL pour révéler la mort cellulaire par apoptose.

### Validation des méthodes

### Contrôle négatif et contrôle positif des expériences

Le contrôle négatif a été fourni par une lame de rétine provenant d'un rat du groupe contrôle « NMDA+DMSO » (groupe 6), sur laquelle, il n'y a pas eu d'enzyme TUNEL déposée lors de la préparation des lames à la méthode TUNEL (mais seulement la solution label TUNEL). L'absence de noyau (marqués au DAPI) TUNEL positif, et donc apoptotiques, valide les expériences. (Fig. 1).

Le contrôle positif permet de vérifier que la méthode TUNEL a bien fonctionné. Les cellules superficielles de l'épithélium cornéen de chaque coupe, parmi lesquelles des cellules apoptotiques sont toujours présentes (cellules desquamantes), ont été utilisées comme contrôle positif. Le marquage TUNEL positif (Fig. 2B) corrèle bien avec des noyaux de l'épithélium cornéen (Fig. 2A), il s'agit donc de noyaux de cellules apoptotiques marqués. La méthode TUNEL est ainsi validée.

### Dégénérescence des cellules rétiniennes induite par le NMDA

24h après l'injection intra-vitréenne de NMDA (rats du groupe 4), de nombreuses cellules TUNEL positif sont visibles dans la couche nucléaire interne (Figure 3B et C). Le NMDA détériorant la rétine de la surface vers la profondeur, cela démontre donc le stade avancé de la dégénérescence de la rétine induite par le NMDA.

### Effet du DMSO sur les cellules rétiniennes (saines ou en dégénérescence)

Le marquage TUNEL effectué après traitement par du DMSO seul (rats du groupe 5) est similaire au marquage des contrôles négatifs, attestant de l'absence de toxicité du DMSO sur des cellules saines de rétines à la concentration de l'expérience (Figure 4A et B).

Les marquages TUNEL effectués après traitement par du DMSO et du NMDA (rats du groupe 6) et après traitement par du NMDA seul (rats du groupe 4) montrent une quantité équivalente de cellules apoptotiques (Figure 4C et D).

Ces résultats attestent de l'absence d'effet toxique ou bénéfique du DMSO sur la neurodégénérescence induite par le NMDA.

### Résultats

Les coupes de rétine obtenues à partir des 6 groupes de rats et marquées au DAPI et au TUNEL ont été observées par microscope et le nombre moyen de cellules apoptotiques (c'est-à-dire TUNEL positif) par champ a été déterminé pour chaque groupe. Ce résultat est présenté sur l'histogramme de la Figure 5.

Ces résultats illustrent tout d'abord l'absence d'effet toxique ou bénéfice du DMSO et la dégénérescence effective des cellules rétiniennes induite par le NMDA.

Les quantités de cellules apoptotiques observées dans les groupes ayant bénéficié d'une injection simultanée de glibenclamide et de NMDA sont diminuées de 82 à 86 % par rapport à la quantité de cellules apoptotiques observées dans les groupes soumis à une injection de NMDA en présence de DMSO.

Ces résultats démontrent donc que l'injection de glibenclamide permet de diminuer de manière significative la dégénérescence des cellules rétiniennes induite par le NMDA.

### Exemple 2 : effet du glibenclamide sur la dégénérescence rétinienne selon les différentes voies d'administration

### Protocole expérimental

8 groupes de 2 rats ont été utilisés. Un traitement identique a été administré aux deux yeux de chaque animal. Trois voies d'administration du glibenclamide ont été testées, la voie intra-vitréenne (injection de 1 ng ou 0,01 ng dans chaque oeil), la voie orale (10 mg/kg de poids corporel de glibenclamide) et la voie sous-conjonctivale (injection de 1 ng de glibenclamide dans chaque oeil).

La solution mère de glibenclamide pour les injections intra-vitréennes et sous-conjonctivales a été obtenue en diluant 4,5 mg de glibenclamide dans 300 µl de DMSO pur. Cette solution a ensuite été congelée. Les solutions de glibenclamide aux différentes concentrations pour les injections intra-vitréennes et sous-conjonctivales ont été obtenues par dilutions extemporanées dans du tampon BSS® (Alcon laboratories).

Les solutions de glibenclamide pour l'administration orale (10 mg/kg) ont été obtenues en dissolvant 8 mg de glibenclamide dans 300 µl de DMSO et 1030 µl d'eau de façon à obtenir une solution contenant 6 mg/ml de glibenclamide. L'administration est réalisée au moyen d'une seringue.

Le NMDA a été dilué dans de l'eau et tamponné au TRIS pH 7.

Le protocole expérimental comprenait huit groupes de rats tels que définis ci-dessous
Groupe 1 : (n=2, 4 yeux), injection intra-vitréenne de 6µL d'une solution comprenant 45 nM de NMDA et 1 ng de glibenclamide.
Groupe 2 : (n=2, 4 yeux), injection intra-vitréenne de 6µL d'une solution comprenant 45 nM de NMDA et 0,01 ng de glibenclamide.
Groupe 3 : (n=2, 4 yeux), injection intra-vitréenne de 6µL d'une solution comprenant 45 nM de NMDA et 260mM de DMSO.
Groupe 4 : (n=2, 4 yeux), administration par voie orale de 10 mg/kg de poids corporel de glibenclamide et injection simultanée intra-vitréenne de 6µL d'une solution de 45 nM NMDA. Une solution de 7% glucose est laissée à disposition à volonté pour éviter une éventuelle hypoglycémie.
Groupe 5 : (n=2, 4 yeux), administration par voie orale d'une solution de DMSO 260 mM et injection simultanée intra-vitréenne de 6 µL d'une solution de 45 nM NMDA. Une solution de 7% glucose est laissée à disposition à volonté.
Groupe 6 : (n=2, 4 yeux), injection sous-conjonctivale de 50 µL d'une solution comprenant 1 ng de glibenclamide et injection simultanée intra-vitréenne de 6 µL d'une solution de 45 nM NMDA.
Groupe 7 : (n=2, 4 yeux), injection sous-conjonctivale de 50 µL d'une solution de DMSO 260 mM et injection simultanée intra-vitréenne de 6 µL d'une solution de 45 nM NMDA.
Groupe 8 : (n=2, 4 yeux), pas de traitement.

18h après l'administration de NMDA et glibenclamide, les rats ont été euthanasiés et les yeux ont été immédiatement prélevés. La fixation des yeux a été réalisée durant 4 heures dans une solution de 4% paraformaldéhyde et 10 % sucrose. Après fixation, les yeux ont ensuite été plongés durant une nuit à 4°C dans une solution de 15% sucrose dans du tampon phosphate.

Les yeux ont ensuite subi une étape d'inclusion en OCT, une étape de coupe en congélation au cryostat (10µm d'épaisseur) et une étape de marquage des coupes par la méthode TUNEL pour révéler la mort cellulaire par apoptose.

### Résultats

Les méthodes utilisées dans cet exemple ont préalablement été validées selon le protocole décrit dans l'exemple 1 (cf. validation des méthodes).

### Administration intra-vitréenne du glibenclamide

L'injection intra-vitréenne de 1 ng (rats du groupe 1) ou de 0,01 ng de glibenclamide (rats du groupe 2) permet de diminuer de manière significative le nombre de cellules apoptotiques dans les rétines dont la dégénérescence est induite par le NMDA (Figure 6). Grâce aux marquages de ces rétines, il a également été observé que l'injection de 1 ou 0,01 ng de glibenclamide permettait de conserver une parfaite organisation des couches rétiniennes (Figure 7B et C) contrairement à ce qui est observé dans les rétines dégénérescentes (Figure 7A).

Ces résultats démontrent que le glibenclamide réduit significativement la dégénérescence et la déstructuration des couches rétiniennes induite par le NMDA, et ce même à très faible dose.

### Administration orale du glibenclamide

L'administration de glibenclamide par voie orale et l'injection intra-vitréenne de NMDA ont été pratiquées le même jour (groupe 4). Les résultats obtenus pour les groupes de rats 4 et 5 sont présentés sur la figure 8.

Ces résultats démontrent que l'administration de glibenclamide par voie orale permet de réduire de manière significative la dégénérescence des cellules rétiniennes induite par le NMDA.

### Administration sous-conjonctivale du glibenclamide

L'administration de glibenclamide par voie sous-conjonctivale et l'injection intra-vitréenne de NMDA ont été pratiquées le même jour (groupe 6). Les résultats obtenus pour les groupes de rats 6 et 7 sont présentés sur la figure 9.

Ces résultats démontrent que l'administration de glibenclamide par voie sous-conjonctivale permet de réduire de manière significative la dégénérescence des cellules rétiniennes induite par le NMDA.

### Exemple 3 : Effet préventif du glibenclamide sur la dégénérescence rétinienne induite par NMDA

### Protocole expérimental

3 groupes de 2 rats ont été utilisés. Un traitement identique a été administré aux deux yeux de chaque animal.

L'effet préventif du glibenclamide a été testé en injectant dans le corps vitré de chaque oeil 100 ng ou 100 µg de glibenclamide dilués dans du tampon BSS®, 5 jours avant l'injection intra-vitréenne de NMDA. Le glibenclamide a été dilué dans du tampon BSS et non dans du DMSO afin d'obtenir une suspension avec effet retard, conséquence de la mauvaise solubilité du glibenclamide.

L'administration de 100 µg de glibenclamide a permis de tester l'éventuelle toxicité à haute dose du glibenclamide.

La solution mère de glibenclamide pour les injections intra-vitréennes a été obtenue en diluant 4,5 mg de glibenclamide dans 300 µl de DMSO pur. Cette solution a ensuite été congelée. Les solutions de glibenclamide aux différentes concentrations pour les injections intra-vitréennes ont été obtenues par dilutions extemporanées dans du tampon BSS® (Alcon laboratories).

Le NMDA a été dilué dans de l'eau et tamponné au TRIS pH 7 de façon à obtenir une concentration de 90 nM.

Le protocole expérimental comprenait trois groupes de rats tels que définis ci-dessous
Groupe 1 : (n=2, 4 yeux), J-5 : injection intra-vitréenne de 3µL d'une solution comprenant 100 µg de glibenclamide dans du tampon BSS® ; J0 : injection intra-vitréenne de 3µL d'une solution de 90 nM de NMDA
Groupe 2 : (n=2, 4 yeux), J-5 : injection intra-vitréenne de 3µL d'une solution comprenant 100 ng de glibenclamide dans du tampon BSS® ; J0 : injection intra-vitréenne de 3µL d'une solution de 90 nM de NMDA
Groupe 3 : (n=2, 4 yeux), J-5 : injection intra-vitréenne de 3µL d'une solution de tampon BSS® ; J0 : injection intra-vitréenne de 3µL d'une solution de 90 nM de NMDA

18h après l'administration de NMDA et glibenclamide, les rats ont été euthanasiés et les yeux ont été immédiatement prélevés. La fixation des yeux a été réalisée durant 4 heures dans une solution de 4% paraformaldéhyde et 10 % sucrose. Après fixation, les yeux ont ensuite été plongés durant une nuit à 4°C dans une solution de 15% sucrose dans du tampon phosphate.

Les yeux ont ensuite subi une étape d'inclusion en OCT, une étape de coupe en congélation au cryostat (10µm d'épaisseur) et une étape de marquage des coupes par la méthode TUNEL pour révéler la mort cellulaire par apoptose.

### Résultats

Les méthodes utilisées dans cet exemple ont préalablement été validées selon le protocole décrit dans l'exemple 1 (cf. validation des méthodes).

L'injection intra-vitréenne de 100 µg (groupe 1) ou 100 ng (groupe 2) de glibenclamide 5 jours avant une injection intra-vitréenne de NMDA permet de protéger de façon significative les cellules rétiniennes d'une dégénérescence induite par le NMDA (Figure 10).

De plus, l'effet protecteur du glibenclamide administré à forte dose (100 µg) démontre l'absence totale de toxicité de cette molécule vis-à-vis des cellules de la rétine.

### Conclusion

L'injection de NMDA induit la neurodégénérescence des cellules de la rétine 18 et 24h après l'injection. Le NMDA agit tout d'abord sur la couche ganglionnaire qui est la plus superficielle et au contact du corps vitré, puis sur les couches plus profondes avant d'atteindre les photorécepteurs. 18h comme 24h après l'injection, le NMDA a déjà agi sur les cellules de la couche ganglionnaire et induit l'apoptose des cellules de la couche nucléaire interne.

Les inventeurs ont démontré au travers de ces différents exemples que l'administration de glibenclamide par voie intra-vitréenne, orale ou sous-conjonctivale permettait de réduire de manière significative l'apoptose rétinienne induite par le NMDA.

Ils ont également montré que l'administration préventive de glibenclamide plusieurs jours avant l'injection de NMDA permettait de prévenir efficacement la dégénérescence des cellules rétiniennes.

### REFERENCES BIBLIOGRAPHIQUES

Celebi S, Dilsiz N, Yilmaz T, Kukner AS. Effects of minelatonin, vitamin E and octreotide on lipid peroxidation during ischemia-reperfusion in the guinea pig 15 retina. Eur J Ophthalmol. 2002 Mar-Apr;12(2):77-83
Chao HM, Osborne NN. Topically applied clonidine protects the rat retina from ischaemia/reperfusion by stimulating alpha(2)-adrenoceptors and not by an action on imidazoline receptors. Brain Res. 2001 Jun 15;904(1):126-36.
Crisanti P, Laplace O, Lecain E, Jonet L, Jeanny JC, Omri B. The role of PKCζ in NMDA-induced retinal ganglion cell death: Prévention by aspirin. Apoptosis 2006; 11:983-991.
Donello JE, Padillo EU, Webster ML, Wheeler LA, Gil DW. alpha(2)Adrenoceptor agonists inhibit vitreal glutamate and aspartate accumulation and preserve retinal function after transient ischemia. J Pharmacol Exp Ther.2001 Jan;296(1):216-23.
Fontaine V, Mohand-Said S, Hanoteau N, Fuchs C, Pfizenmaier K, Eisel U. Neurodegenerative and neuroprotective effects of tumor Necrosis factor (TNF) in retinal ischemia: opposite roles of TNF receptor 1 and TNF receptor 2. J Neurosci. 2002 Apr 1;22(7).
Lam TT, Siew E, Chu R, Tso MOM. Ameliorative effect of MK801 on retinal ischemia. J Ocular Pharmacol Ther 1997 ; 13 : 129-37.
Liss B, Roeper J. Molecular physiology of neuronal K-ATP channels. Mol. Membr. Biol 2001;18:117-127.
Nistico R, Piccirilli S, Sebastianelli L, Nistico G, Bernardi G, Mercuri NB. The blockade of K (+)-ATP channels has neuroprotective effects in an in vitro model of brain ischemia. Int Rev Neurobiol 2007; 82:383-95.
Osborne NN, Schwarz M, Pergande G. Protection of rabbit retina from ischemic injury by flupirtine. Invest Ophthalmol Vis Sci 1996 ; 37 : 274-80.
Pitlik S, Manor RS, Lipshitz I, Perry G, Rosenfeld J. Transient retinal ischaemia induced by nifedipine. Br Med J (Clin Res Ed). 1983 Dec 17;287(6408):1845-6.
Sherry DM, Townes-Anderson E. Rapid glutamatergic alterations in the neural retina induced by retinal detachment. Invest Ophthalmol Vis Sci. 2000 Aug;41(9):2779-90
Simard JM, Chen M, Tarasov KV, Bhatta S, Ivanova S, Melnitchenko L, Tsymbalyuk N, West GA, Gerzanich V. Newly expressed SUR1-regulated NC(Ca-ATP) channel mediates cerebral edema after ischemic stroke. Nat Med. 2006 Apr;12(4):433-40
Simard JM, Woo SK, Bhatta S, Gerzanich V. Drug acting on SURI to treat CNS ischemia and trauma. Curr Opin Pharmacol. 2008 Feb; 8(1): 42-9.
Szabo ME, Haines D, Garay E, Chiavaroli C, Farine JC, Hannaert P, Berta A, Garay RPL. Antioxidant properties of calcium dobesilate in ischemic/reperfused diabetic rat retina. Eur J Pharmacol. 2001 Oct 5;428(2):277-86.
Tsukahara Y, Blair NP, Spellman Eappen DC, et al. Ketamine suppresses ischemic injury in the rabbit retina. Invest Ophthalmol Vis Sci 1992 ; 33 : 1822-5.
Vorwerk CK, Lipton SA, Zurakowski D, et al. Chronic low-dose glutamate is toxic to retinal ganglion cells. Toxicity blocked by memantine. Invest Ophthalmol Vis Sci 1996 ; 37 : 1619-24.
Yoon YH, Marmor MF. Dextromethorphan protects retina against ischemic injury in vivo. Arch Ophthalmol 1989 ; 107 : 409-11.

## Revendications

1. Inhibiteur des canaux ioniques régulés par la sous-unité SUR pour une utilisation dans le traitement et/ou la prévention d'une pathologie associée à l'ischémie rétinienne et/ou à une excitotoxicité rétinienne, dans lequel ledit inhibiteur est sélectionné dans le groupe constitué du glibenclamide, l'acétohexamide, le carbutamide, le glibornuride, le chlorpropamide, le tolbutamide, le tolazamide, le glipizide, le gliquidone, le glyclopyramide, le glisoxepide et le glimepiride, et dans lequel la pathologie associée à l'ischémie rétinienne et/ou à une excitotoxicité rétinienne est sélectionnée dans le groupe constitué du glaucome, des neuropathies optiques glaucomateuses sans hypertonie, de la dégénérescence maculaire liée à l'âge, des inflammations intraoculaires aigues ou chroniques (les uvéites, uvéo-rétinites, les choroïdites), des neuropathies optiques ischémiques ou toxiques, de l'endophtalmie, des rétinites infectieuses, de la rétinopathie diabétique, de la rétinopathie du prématuré, de la rétinopathie ischémique proliférante, des rétinites pigmentaires, des rétinopathies associées à une hémoglobinopathie, d'une photo-dégénérescence, d'un décollement de la rétine, des pathologies vasculaires rétiniennes et choroïdiennes (sténoses, thromboses et occlusions vasculaires), des hémorragies rétiniennes et/ou choroïdiennes, de la myopie et des dégénérescences rétiniennes héréditaires ou acquises.

2. Inhibiteur pour une utilisation selon la revendication 1, dans lequel ledit inhibiteur est le glibenclamide.

3. Inhibiteur pour une utilisation selon la revendication 1 ou 2, dans lequel ledit inhibiteur est administré en combinaison avec une autre substance active, de façon simultanée ou séquentielle.

4. Inhibiteur pour une utilisation selon l'une quelconque des revendications 1 à 3, dans lequel la pathologie associée à l'ischémie rétinienne et/ou à une excitotoxicité rétinienne est sélectionnée dans le groupe constitué du glaucome, la dégénérescence maculaire liée à l'âge, la rétinopathie diabétique, la rétinopathie du prématuré, la rétinopathie ischémique proliférante et une rétinite pigmentaire.

5. Inhibiteur pour une utilisation selon l'une quelconque des revendications 1 à 4, dans lequel le sujet à traiter est un humain sélectionné dans le groupe constitué par un nourrisson, un enfant et un adulte.

6. Inhibiteur pour une utilisation selon l'une quelconque des revendications 1 à 5, dans lequel le sujet à traiter est un animal sélectionné dans le groupe constitué d'un chien, un chat, un cheval, une vache, un mouton, un cochon et un primate non-humain

7. Inhibiteur pour une utilisation selon l'une quelconque des revendications 1 à 6, dans lequel ledit inhibiteur est administré par voie intra-vitréenne, par voie sous-conjonctivale, par voie orale, par instillation topique, par voie péri-oculaire et intraoculaire ou par voie parentérale.

## Patentansprüche

1. Hemmer von Ionenkanälen, die von der SUR-Untereinheit reguliert werden, für eine Verwendung in der Behandlung und/oder Prävention einer Krankheit, die mit der retinalen Ischämie und/oder mit einer retinalen Exzitotoxizität assoziiert ist, wobei besagter Hemmer aus der Gruppe ausgewählt ist, bestehend aus Glibenclamid, Acetohexamid, Carbutamid, Glibomurid, Chlorpropamid, Tolbutamid, Tolazamid, Glipizid, Gliquidon, Glyclopyramid, Glisoxepid und Glimepirid, und wobei die Krankheit, die mit der der retinalen Ischämie und/oder mit einer retinalen Exzitotoxizität assoziiert ist, aus der Gruppe ausgewählt ist, bestehend aus Glaukom, glaukomatösen optischen Neuropathien ohne Hypertonie, altersbedingter Makulatadegeneration, chronischen oder akuten intraokularen Entzündungen (Uveitis, Uveoretinitis, Choroiditis), toxischen oder ischämischen optischen Neuropathien, Endophthalmitis, infektiösen Retinitiden, diabetischer Retinopathie, Frühgeborenenretinopathie, proliferativer ischämischer Retinopathie, Retinitis pigmentosa, mit Hämoglobinopathie assoziierten Retinopathien, Photodegeneration, Netzhautablösung, choroidalen und retinalen Gefäßkrankheiten (Stenosen, Thrombosen und Gefäßverschlüssen), choroidalen und/oder retinalen Hämorrhagien, Myopie und erworbenen oder erblichen Netzhautdegenerationen.

2. Hemmer für eine Verwendung gemäß Anspruch 1, wobei besagter Hemmer Glibenclamid ist.

3. Hemmer für eine Verwendung gemäß Anspruch 1 oder 2, wobei besagter Hemmer in Kombination mit einer weiteren aktiven Substanz gleichzeitig oder nacheinander verabreicht wird.

4. Hemmer für eine Verwendung gemäß einem der Ansprüche 1 bis 3, wobei die Krankheit, die mit der retinalen Ischämie und/oder mit einer retinalen Exzitotoxizität assoziiert ist, aus der Gruppe ausgewählt ist, bestehend aus Glaukom, altersbedingter Makulatadegeneration, diabetischer Retinopathie, Frühgeborenenretinopathie, proliferativer ischämischer Retinopathie und Retinitis pigmentosa.

5. Hemmer für eine Verwendung gemäß einem der Ansprüche 1 bis 4, wobei das zu behandelnde Lebewesen ein Mensch ist, ausgewählt aus der Gruppe, bestehend aus einem Säugling, einem Kind und einem Erwachsenen.

6. Hemmer für eine Verwendung gemäß einem der Ansprüche 1 bis 5, wobei das zu behandelnde Lebewesen ein Tier ist, ausgewählt aus der Gruppe, bestehend aus einem Hund, einer Katze, einem Pferd, einer Kuh, einem Schaf, einem Schwein und einem nicht-humanen Primaten.

7. Hemmer für eine Verwendung gemäß einem der Ansprüche 1 bis 6, wobei besagter Hemmer intravitreal, subkonjunktival, oral, mittels topischer Instillation, periokular und intraokular oder parenteral verabreicht wird.

## Claims

1. Blocker of ion channels regulated by the SUR subunit for a use in the treatment and/or prevention of a disease associated with retinal ischemia and/or retinal excitotoxicity, wherein said blocker is selected from the group consisting of glibenclamide, acetohexamide, carbutamide, glibomuride, chlorpropamide, tolbutamide, tolazamide, glipizide, gliquidone, glyclopyramide, glisoxepide and glimepiride, and wherein the disease associated with retinal ischemia and/or retinal excitotoxicity is selected from the group consisting of glaucoma, glaucomatous optic neuropathies without hypertonia, age-related macular degeneration, acute or chronic intraocular inflammations (uveitis, uveoretinitis, choroiditis), ischemic or toxic optic neuropathies, endophthalmitis, infectious retinitis, diabetic retinopathy, retinopathy of prematurity, ischemic retinitis proliferans, retinitis pigmentosa, retinopathies associated with a hemoglobinopathy, photodegeneration, retinal detachment, retinal and choroidal vascular disorders (stenosis, thrombosis and vascular occlusions), retinal and/or choroidal hemorrhage, myopia and hereditary or acquired retinal degeneration.

2. Blocker for use according to claim 1, wherein said blocker is glibenclamide.

3. Blocker for use according to claim 1 or 2, wherein said blocker is administered in combination with another active substance, simultaneously or sequentially.

4. Blocker for use according to any of claims 1 to 3, wherein the disease associated with retinal ischemia and/or retinal excitotoxicity is selected from the group consisting of glaucoma, age-related macular degeneration, diabetic retinopathy, retinopathy of prematurity, ischemic retinitis proliferans and retinitis pigmentosa.

5. Blocker for use according to any of claims 1 to 4, wherein the subject to be treated is a human being selected from the group consisting of an infant, a child and an adult.

6. Blocker for use according to any of claims 1 to 5, wherein the subject to be treated is an animal selected from the group consisting of a dog, a cat, a horse, a cow, a sheep, a pig and a non-human primate.

7. Blocker for use according to any of claims 1 to 6, wherein said blocker is administered by the intravitreal route, by the subconjunctival route, by the oral route, by topical instillation, by the periocular and intraocular route or by the parenteral route.
